# EUROPÄISCHE PATENTSCHRIFT

(11) **EP 3 069 743 B1**
(45) Veröffentlichungstag und Bekanntmachung des Hinweises auf die Patenterteilung: **07.08.2019**
(21) Anmeldenummer: 16160153.9
(22) Anmeldetag: 14.03.2016
(51) Int. Cl.: A61M 1/16

(54) **BLUTBEHANDLUNGSGERÄT MIT SEPARATEM TÜRABTEIL**
BLOOD PROCESSING DEVICE WITH SEPARATE DOOR COMPARTMENT
APPAREIL DE TRAITEMENT DU SANG COMPRENANT UNE PORTE DOTÉ D'UN COMPARTIMENT

(30) Priorität: 17.03.2015 DE 102015103937
(43) Veröffentlichungstag der Anmeldung: 21.09.2016
(73) Patentinhaber: B. Braun Avitum AG, 34212 Melsungen (DE)
(72) Erfinder: Iske, Andreas, 34320 Söhrewald (DE); Stenzel, Bruno, 34346 Hann. Münden (DE)
(74) Vertreter: Winter, Brandl, Fürniss, Hübner, Röss, Kaiser, Polte - Partnerschaft mbB

(56) Entgegenhaltungen:
- EP-A2- 0 432 146
- US-A1- 2004 064 080
- US-A1- 2011 098 635
- US-A1- 2014 112 828

## Beschreibung

Die vorliegende Erfindung betrifft ein Blutbehandlungsgerät mit separatem, Türintegriertem Aufnahmeabteil für Gerätekomponenten und insbesondere ein Gehäuse für ein Blutbehandlungsgerät. Einem Blutbehandlungsgerät der erfindungsgemäßen Gattung ist auch ein Dialysegerät zuzuordnen.

### Hintergrund der Erfindung

Bei einem Blutbehandlungsgerät, insbesondere einem Dialysegerät, ist unter anderem der Schutz eines Bedieners und/oder eines Patienten wichtig. Wenn beispielsweise das Dialysegerät im Desinfektionsmodus betrieben wird, werden insbesondere die Dialysierflüssigkeitsfilter sehr heiß und da die Dialysierflüssigkeitsfilter üblicherweise außen an einem Gehäuse des Dialysegeräts angebracht sind, besteht für den Bediener und/oder den Patienten eine erhöhte Verletzungsgefahr. Darüber hinaus besteht bei außen angebrachten Dialysierflüssigkeitsfiltern neben einem erhöhten Platzbedarf die Gefahr, dass bei der Gerätehandhabung Schlauchkupplungen versehentlich von den Dialysierflüssigkeitsfiltern gelöst werden und eine Leckage verursacht wird. Auch ist beispielsweise bei einem Versetzen des Dialysegeräts an einen anderen Ort eine Beschädigung der außen angebrachten Dialysierflüssigkeitsfilter möglich. Zudem lässt sich eine Leckage bei außen angebrachten Dialysierflüssigkeitsfiltern nicht oder nur sehr aufwändig erfassen und eine einen Patienten gefährdende Ultrafiltrationsabweichung würde unentdeckt bleiben.

Dabei ist eine einfache Unterbringung der Dialysierflüssigkeitsfilter in dem Gehäuse des Dialysegeräts keine praktikable Lösung, da die Dialysierflüssigkeitsfilter beispielsweise bei der Bedienung und bei der Wartung leicht zugänglich sein sollen. Zudem würden ins Gehäuseinnere verlegte Dialysierflüssigkeitsfilter den dort zur Verfügung stehenden Platz verringern und die Zugänglichkeit anderer darin befindlicher Komponenten erschweren.

### Stand der Technik

Aus der DE 10 2009 04 50 95 A1 ist ein Gehäuse für ein Blutbehandlungsgerät mit Verschlussklappe bekannt. Bei diesem Gehäuse ist eine Öffnung mittels einer Verschlussklappe verschließbar, an deren Innenseite ein extrakorporales Blutbehandlungsmodul vorgesehen ist. Dabei ist ein Koppelelement über eine erste Drehachse gegen eine Gehäusewand drehbar gelagert und die Verschlussklappe ist über eine zweite Drehachse gegen das Koppelelement drehbar gelagert. Dadurch soll erreicht werden, dass in einer herausgeklappten Stellung der Verschlussklappe sowohl das extrakorporale Blutbehandlungsmodul zugänglich ist, als auch die außen an der Verschlussklappe befindlichen Anzeigen und Bedienelemente für das extrakorporale Blutbehandlungsmodul einsehbar beziehungsweise zugänglich sind.

Nachteilig dabei ist, dass auch wenn lediglich ein Zugang zu anderen Komponenten im Gehäuseinneren erforderlich ist, die Verschlussklappe geöffnet werden muss und so auch das extrakorporale Blutbehandlungsmodul zumindest teilweise nach außen geschwenkt wird, selbst wenn daran gar nicht gearbeitet werden soll. Unabsichtliche Veränderungen an dem extrakorporalen Blutbehandlungsmodul, insbesondere an Anschlüssen daran, sind somit erst ermöglicht. Zudem behindert das extrakorporale Blutbehandlungsmodul auf diese Weise einen freien Zugang zu anderen im Gehäuseinneren vorhandenen Weiterer relevanter Stand der Technik ist offenbart in Dokumenten EP0432146 A2, US2004/0064080 A1 und US2014/0112828 A1.

### Kurzbeschreibung der Erfindung

Der Erfindung liegt angesichts dieses bekannten Stands der Technik die grundsätzliche Aufgabe zugrunde, ein Blutbehandlungsgerät, vorzugsweise Dialysegerät anzugeben, das einen verbesserten Schutz einer darin oder daran aufzunehmenden Gerätekomponente bei leichter Zugänglichkeit derselben ermöglicht.

Eine Gerätekomponente im Sinne der Erfindung meint einen in oder an dem Gehäuse des erfindungsgemäßen Blutbehandlungsgeräts anzuordnenden Bestandteil und schließt zunächst auch Gerätezubehör mit ein. Vorzugsweise sind unter Gerätekomponenten jene Bestandteile des Blutbehandlungsgeräts zu verstehen, welche aus Gründen der vereinfachten Handhabung in der Regel außen am Gehäuse angeordnet sind, wie beispielsweise Dialysefilter, geräteseitige Zu- und/oder Abführschläuche, Kartuschen die Zusätze zur Zubereitung der Dialysierflüssigkeit beinhalten, etc.

Eine weitere Aufgabe der Erfindung ist es, ein Blutbehandlungsgerät, vorzugsweise Dialysegerät mit einem Gehäuse zur Verfügung zu stellen, bei dem eine normalerweise außerhalb des Gehäuses angeordnete Gerätekomponente des Blutbehandlungsgeräts vor einer unabsichtlichen Veränderung, insbesondere vor einer Beschädigung, geschützt und trotzdem gut zugänglich ist.

Schließlich ist es Ziel der Erfindung, die Zugänglichkeit jener Gerätekomponenten, die bekanntermaßen innerhalb des Geräts angeordnet sind, auch bei Umsetzung des vorliegenden Erfindungsgedankens nicht zu verschlechtern.

Die Aufgaben werden durch ein Gehäuse eines Blutbehandlungsgeräts mit den Merkmalen nach Anspruch 1 gelöst, und durch ein Blutbehandlungsgerät, vorzugsweise Dialysegerät mit den Merkmalen nach Anspruch 13. Bevorzugte Ausführungsformen der vorliegenden Erfindung sind Gegenstand der jeweiligen Unteransprüche.

Der Erfindung liegt der allgemeine Gedanke zugrunde, jene Gerätekomponenten insbesondere Dialysefilter ebenfalls einzuhausen, welche bisher im Stand der Technik außerhalb des Gerätegehäuses angeordnet sind. Jedoch sollte die Einhausung so erfolgen, dass das Volumen innerhalb des Gerätegehäuses nicht Übergebühr eingeschränkt wird und auch der Zugang zu den normalerweise innerhalb des Gerätegehäuses angeordneten Gerätekomponenten wie Pumpen, Wärmetauscher, Sensoren. etc. nicht erschwert wird. Dieser Gedanke lässt sich konzeptionell dadurch umsetzen, indem die normalerweise außerhalb des Gerätegehäuses angeordneten Gerätekomponenten in einem Gehäuseabteil separat zu dem Gerätegehäuse jedoch vorzugsweise zumindest teilweise innerhalb des Gerätegehäuses aufgenommen sind, welches vom Gerätegehäuse distanziert/beabstandet werden kann, um so Zugangsraum für die darin verbleibenden Gerätekomponenten zu schaffen.

Im Prinzip macht sich die vorliegende Erfindung das Konstruktionskonzept beispielsweise eines allgemein bekannten Kühlschranks zunutze, wie er in herkömmlichen Küchen zu finden ist. Dort werden beispielsweise an der Innenseite der Kühlschranktür separate Lagerabteile für zerbrechliche Nahrungsmittel oder - behälter gebildet, die durch eine Innentür oder Klappe vom übrigen Kühlschrankinnenraum getrennt sind und die zusammen mit der Kühlschranktür vom Kühlschrankinneren weggeschwenkt werden, um den Zugang zum Kühlschrankinnenraum zu erweitern.

Diesem Konstruktionskonzept folgend sieht die Erfindung zur Lösung der gestellten Aufgabe eine Tür-in-Tür Anordnung vor, wonach eine (ohnehin vorhandene) Gehäusetür zur Freigabe/Verschließung eines Gerätegehäusezugangs mit einem Hohlraum/Aufnahmeabteil ausgebildet ist, der von einer weiteren Abteiltür oder - klappe (Außen- oder Innentür) verschließbar ist, die wiederum an der Gehäusetür vorzugsweise an deren Außenseite angeschlagen bzw. anscharniert ist. D.h. die Gerätegehäusetür dient gleichzeitig als Aufnahmeabteil für die normalerweise außerhalb des Gerätegehäuses angeordneten Gerätekomponenten vorzugsweise Filter/Filterkartuschen, sodass diese zwangsläufig mit der Gerätegehäusetür vom Blutbehandlungsgerät /Dialysegerät weggeschwenkt/beabstandet werden, wenn ein Zugang in das Gehäuseinnere geöffnet werden soll.

Trotz dieser separaten Einhausung in der Gerätegehäusetür verbleiben somit sowohl der Zugang zum Gehäuseinneren wie auch der Zugang zu den Gehäusetür-eingehausten Gerätekomponenten nahezu unverbaut. Damit können die normalerweise außerhalb des Gerätegehäuses angeordneten Gerätekomponenten mit nur geringfügigem Mehraufwand nämlich das (außenseitige) Öffnen der Abteiltür oder -klappe (sowohl bei geöffneter wie auch verschlossener Gerätegehäusetür) beispielsweise gewechselt werden. Jedoch liegen sie geschützt und das Gerätegehäuseinnere wird nicht Übergebühr eingeengt. Darüber hinaus kann der in der Gerätegehäusetür ausgebildete Hohlraum/Abteil zumindest teilweise oder vollständig in das Gehäuseinnere ragen, sodass die Außenabmessung des Blutbehandlungsgeräts, vorzugsweise Dialysegeräts gegenüber bekannten Geräten im Wesentlichen nicht vergrößert werden muss. Da der Hohlraum/Abteil mit der Gerätegehäusetür bei deren Öffnen mitgeschwenkt wird, bleibt der Zugang in das Gehäuseinnere unverbaut.

Konkreter hat ein erfindungsgemäßes Gehäuse eines Blutbehandlungsgeräts, vorzugsweise Dialysegeräts wenigstens eine Wand und eine Öffnung, die in der Wand vorgesehen ist. Weiter hat das Gehäuse wenigstens eine Gehäusetür vorzugsweise eine Wartungs-, oder Belieferungstür, mittels der die Öffnung verschließbar ist.

Erfindungsgemäß weist die Tür, oder eine weitere Wand, einen Bauraum/Abteil für eine Gerätekomponente auf, der mittels einer vorzugsweise außenseitig an der Gehäusetür angeschlagenen Zusatztür (Tür-in-Tür) verschließbar ist.

Ein Vorteil des erfindungsgemäßen Gerätegehäuses ist, dass die in dem separaten Bauraum/Türabteil angeordnete(n) Gerätekomponente(n) insbesondere des Dialysegeräts vor Beschädigung geschützt ist/sind. Ein weiterer Vorteil ist, dass ein Bediener und/oder ein Patient vor Verletzungen durch eine in dem Bauraum/Abteil angeordnete Gerätekomponente geschützt ist. Dabei ist die in dem Bauraum/Abteil angeordnete wenigstens eine Gerätekomponente trotzdem noch für eine Bedienung und/oder Wartung/Austausch leicht zugänglich. Zudem hat das erfindungsgemäße Gerätegehäuse einen verringerten Platzbedarf, weil die in dem Bauraum/Abteil angeordnete(n) Gerätekomponente(n) so gut wie keinen Platz außerhalb des Gehäuses beansprucht.

Nachfolgend genannte Weiterbildungen für die Gerätetür sollen sinngemäß auch für die weitere Wand gelten.

Vorteilhafterweise ist der Bauraum/Abteil nach außen offen und mittels der Zusatztür (Abteiltür) von außen verschließbar. Dies erleichtert die Zugänglichkeit der darin angeordneten Gerätekomponente auch bei geschlossener Gerätegehäusetür.

Der Bauraum/Abteil kann als eine Vertiefung, insbesondere eine Mulde, ausgebildet sein. Weiter kann der Bauraum/Abteil zumindest abschnittweise durch eine gitterförmige Wand vom übrigen Gehäuseinneren separiert sein.

Vorteilhafterweise sind die Gerätegehäusetür und die Zusatztür jeweils unabhängig voneinander von außen bedienbar. Im Fall, dass die Gerätegehäusetür (nachfolgend der Einfachheit halber auch Gehäusetür bezeichnet) beispielsweise zum Zweck einer Wartung einer in dem Gehäuse untergebrachten anderen Gerätekomponente geöffnet ist oder geöffnet werden muss, kann die Zusatztür geschlossen bleiben und so bleibt ihre mehrfache Schutzfunktion für die beziehungsweise vor der in dem Bauraum/Abteil angeordneten Gerätekomponente weiter erhalten. Zudem lässt sich die Zusatztüre für eine Bedienung und/oder die Wartung der in dem Bauraum/Abteil angeordneten Gerätekomponente öffnen, ohne die Gerätetüre öffnen zu müssen. Dies erleichtert einerseits die Zugänglichkeit der in dem Bauraum/Abteil angeordneten Gerätekomponente und schützt andererseits weiterhin die beziehungsweise vor der in dem Gerätegehäuse untergebrachten anderen Gerätekomponente(n).

Vorteilhafterweise ist die Zusatztür, insbesondere außen, an der Gerätetür angeordnet. Somit ist die Zusatztür jederzeit leicht zugänglich und bedienbar, ob bei geöffneter oder geschlossener Gerätetür.

Vorteilhafterweise sind/ist die Gerätetür und/oder die Zusatztür als eine nach außen öffnende Schwenktür ausgebildet. Somit schwenkt der an der Gerätetür angeordnete Bauraum/Abteil mit der als nach außen sich öffnenden Schwenktür ausgebildeten Zusatztür aus dem Gerätegehäuse heraus und erhöht somit die Zugänglichkeit anderer in dem Gerätegehäuse untergebrachten Gerätekomponenten, insbesondere derer des Dialysegeräts. Dass die Zusatztür alleine oder ebenfalls als nach außen öffnende Schwenktür ausgebildet ist, gewährleistet weiter den oben erwähnten Vorteil der erleichterten Zugänglichkeit des Bauraums/Abteils und der darin aufzunehmenden Gerätekomponente(n) und der unabhängig voneinander zu öffnenden Gerätetür und Zusatztür.

Weiter vorteilhaft ist es, die Gerätetür und/oder die Zusatztür als eine, insbesondere mehrteilige/mehrgliedrige, Schiebetür auszubilden. Diese Ausgestaltung bietet je nach zur Verfügung stehendem Platzangebot in dem Gehäuse oder der Gehäuseumgebung weitere Vorteile bei der unabhängig voneinander zu öffnenden Gerätetür und Zusatztür beziehungsweise bei der Gewährleistung einer erleichterten Zugänglichkeit des Bauraums/Abteils und der darin aufzunehmenden Gerätekomponente.

Vorteilhafterweise ist ein Sensor, insbesondere ein Positionssensor, vorgesehen, mittels dem eine offene und/oder eine geschlossene Stellung der Gerätetür und/oder der Zusatztür erfassbar ist. Somit ist insbesondere ein unabsichtliches Öffnen der Zusatztür vermeidbar und die oben erwähnte mehrfache Schutzfunktion der Zusatztür weiter sichergestellt. Zudem ist somit ein unabsichtliches Öffnen der Gerätetür vermeidbar und die anderen Gerätekomponenten bleiben durch das erfindungsgemäße Gehäuse geschützt.

Auch ist es denkbar, durch die Anordnung des Sensors beispielsweise ein Öffnen der Gerätetür bei geöffneter Zusatztür zu verhindern.

Vorteilhafterweise ist der Bauraum/Abteil nach innen über die Gerätetür überstehend angeordnet. Auf diese Weise sind Gerätekomponenten mit mehr Platzbedarf in dem Bauraum/Abteil anordenbar.

Der Bauraum/Abteil kann alternativ oder ergänzend dazu nach außen über die Gerätetür überstehend angeordnet sein. Somit sind Gerätekomponenten mit weiter erhöhtem Platzbedarf in dem Bauraum/Abteil anordenbar.

Weiter vorteilhaft ist der Bauraum/Abteil außen etwa bündig mit der Gerätetür angeordnet/ausgebildet, was einen verringerten Außenumfang des erfindungsgemäßen Gehäuses bewirkt.

Weiter kann der Bauraum/Abteil innen und außen etwa bündig mit der Gerätetür angeordnet sein. Bei Gerätekomponenten mit geringerem Platzbedarf ist diese nach innen und außen platzsparende Ausgestaltung des Bauraums/Abteils von Vorteil.

Vorteilhafterweise ist die Zusatztür in ihrer geschlossenen Stellung mit der Gerätetür außen etwa bündig abschließend angeordnet. Diese Anordnung begünstigt weiter eine geringe äußere Baugröße des erfindungsgemäßen Gehäuses.

Vorteilhafterweise ist die Gerätetür als ein, insbesondere mehrteiliges, Profil ausgebildet, was eine kostengünstige Herstellung ermöglicht. Insbesondere ist der Bauraum/Abteil einstückig mit der Gerätetür ausgebildet, was in Bezug auf eine kostengünstige Herstellung der Gerätetür einen weiteren Vorteil bietet. Je nach Anforderung bezüglich beispielsweise Herstellung, Lagerhaltung und/oder Handhabung kann der Bauraum/Abteil gesondert von der Gerätetür ausgebildet an dieser bei Bedarf montiert sein.

Vorteilhafterweise ist die Gerätetür aus Blech oder Kunststoff herstellbar. Diese Werkstoffe sind im klinischen Einsatz erprobt und es existieren problemlose Herstellverfahren dafür.

Vorteilhafterweise ist in dem Bauraum/Abteil oder in der Gerätetür eine Sammeleinrichtung zum Sammeln einer Leckagemenge, insbesondere einer Dialysierflüssigkeit, vorgesehen. Somit ist eine gegebenenfalls auftretende Leckagemenge gezielt auffangbar und sammelbar.

Vorteilhafterweise ist ein Leckagesensor zur Erfassung der Leckagemenge vorgesehen. Insbesondere mit der Sammeleinrichtung ist somit die Leckagemenge sicher detektierbar. Insbesondere im Fall eines Dialysegeräts als das Blutbehandlungsgerät ist somit eine einen Patienten gefährdende Ultrafiltrationsabweichung sicher erfassbar und die dabei nötigen Maßnahmen können unverzüglich eingeleitet werden.

Vorteilhafterweise weist die Sammeleinrichtung eine Bodenfläche oder Auffangschale auf, die den Bauraum/Abteil zumindest abschnittsweise begrenzt. Somit ist weiter gewährleistet, dass eine in dem Bauraum/Abteil auftretende Leckage sicher aufgefangen wird und nicht etwa den Innenraum des erfindungsgemäßen Gehäuses verunreinigt.

Vorteilhafterweise ist die Wand eine Rückwand. Insbesondere im Fall eines Dialysegeräts als das Blutbehandlungsgerät ist somit eine einen Patienten nicht beeinträchtigende Bedienung ermöglicht. Alternativ oder ergänzend dazu kann die Wand eine Seitenwand oder eine Vorderwand sein.

Bei einem Blutbehandlungsgerät, vorzugsweise Dialysegerät mit einem erfindungsgemäßen Gehäuse ist in dem separaten Bauraum/Abteil eine Gerätekomponente des Dialysegeräts angeordnet, die einen Dialysierflüssigkeitsfilter, eine Bicarbonatkartusche, und/oder eine Blutdruckmanschette aufweist.

Vorteilhafterweise weist der Bauraum/Abteil eine Halterung und/oder eine Schnittstelle für die in dem Bauraum enthaltene(n) Gerätekomponente(n) auf. Insbesondere im Fall, dass die Gerätekomponente einen Dialysierflüssigkeitsfilter aufweist, enthält die Schnittstelle beispielsweise eine Schlauchdurchführung oder einen Schlauchanschluss vom beziehungsweise zum Inneren des Gehäuses.

Weitere Merkmale und Vorteile der Erfindung ergeben sich aus der folgenden Beschreibung der bevorzugten Ausführungsform, bei der Bezug genommen wird auf die beigefügte Zeichnung.

### Figurenbeschreibung

Figur 1 zeigt eine Ausführungsform eines erfindungsgemäßen Blutbehandlungsgeräts, vorzugsweise Dialysegeräts mit einem Gerätegehäuse gemäß der vorliegenden Erfindung mit offener Gerätegehäusetür und offener Zusatztüre in der Gerätegehäusetür,
Figur 2 zeigt die Ausführungsform des Dialysegeräts gemäß Figur 1 mit geschlossener Gerätegehäusetür und geschlossener Zusatztür,
Figur 3 zeigt eine geschnittene Ansicht der Gehäusetür des Dialysegeräts gemäß Figur 1 von schräg unten,
Figur 4 zeigt eine geschnittene Unteransicht eines Details der Gehäusetür des Dialysegeräts gemäß Figur 1, und
Figur 5 zeigt eine Ansicht des Bauraums/Abteils des Dialysegeräts gemäß Figur 1 von schräg innen mit durchsichtig dargestellten Wänden des Bauraums/Abteils.

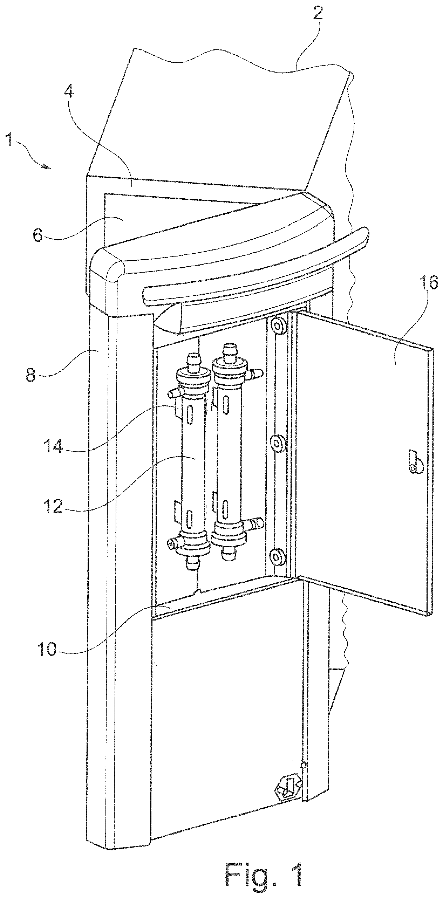

Fig. 1 zeigt ein Ausführungsbeispiel des erfindungsgemäßen Blutbehandlungsgeräts, vorzugsweise Dialysegeräts 1 mit einem Gehäuse 2 gemäß der vorliegenden Erfindung. In einer Wand 4 des Gehäuses 2 ist eine Öffnung 6 vorgesehen und an der Wand 4 ist eine als Schwenktür ausgebildete Gehäusetür 8 seitlich angeschlagen, mit der die Öffnung 6 des Gehäuses 2 verschließbar ist, wobei die Wand 4 vorzugsweise eine Rückwand des Gehäuses 2 ist. Die Gehäusetür 8 enthält/bildet einen türinternen Bauraum/Abteil 10, in dem eine oder mehr Gerätekomponenten untergebracht sind, hier Dialysierflüssigkeitsfilter 12, die jeweils mit einer an der Gerätetür 8 montierten abteilinternen Halterung 14 lösbar befestigt sind. Der Bauraum/Abteil 10 ist als eine nach außen offene Mulde in der Gehäusetür 8 ausgebildet, die mittels einer als seitlich an der Gehäusetür 8 angeschlagenen Schwenktür ausgebildeten Zusatztür 16 (von außen) verschließbar ist. Der Bauraum 10 hat eine oder mehrere (nicht dargestellte) Schnittstellen (Zugänge) wie beispielsweise eine Schlauchdurchführung oder einen Schlauchanschluss vom Inneren des Gehäuses 2 für die Dialysierflüssigkeitsfilter 12. Über die Öffnung 6 sind in dem Gehäuse 2 untergebrachte andere Gerätekomponenten beispielsweise für Wartungszwecke zugänglich. Die Öffnung 6 erstreckt sich hier fast über die gesamte (Rück-)Wand 4, um eine möglichst gute Zugänglichkeit der in dem Gehäuse 2 untergebrachten anderen Gerätekomponenten zu ermöglichen. In einer nicht dargestellten Ausgestaltung kann die Öffnung 6 einen Teil der Wand 4 ausmachen, je nach erforderlicher Zugänglichkeit der in dem Gehäuse 2 untergebrachten anderen Gerätekomponenten und/oder je nach gewünschter Stabilität des Gehäuses 2. Für die Stabilität können aber auch andere übliche Maßnahmen, wie beispielsweise eine Verstärkung der an die Wand 4 angrenzenden (Seiten-)Wände, ergriffen werden.

In einer weiteren nicht dargestellten Ausgestaltung können die Gehäusetür 8 und/oder die daran montierte Zusatztür 16 als eine mehrgliedrige Schiebetür, insbesondere als ein Rollo, ausgebildet sein. Je nach Anforderung und Platzbedarf an dem und um das erfindungsgemäße Dialysegerät 1 kann beispielsweise die Gehäusetür 8 wie dargestellt als rechts angeschlagene Schwenktür ausgebildet sein und die Zusatztür 16 als nach unten öffnendes Rollo (nicht dargestellt) vorgesehen sein, das außen an der Gehäusetür 8 oder innen in der Gehäusetür 8 läuft.

Die Gehäusetür 8 und die Zusatztür 16 sind jeweils unabhängig voneinander von außen bedienbar, so dass beispielsweise bei einer erforderlichen Wartung von in dem Gehäuse 2 untergebrachten anderen Gerätekomponenten lediglich die Gehäusetür 8 geöffnet werden muss und die Zusatztür 16 geschlossen bleiben kann, wodurch die in dem Bauraum/Abteil 10 angeordneten Dialysierflüssigkeitsfilter 12 weiter (auch bei Öffnen der Gehäusetür 8) vor äußeren Einflüssen geschützt bleiben. Ebenso ist dadurch eine Bedienung und/oder die Wartung der in dem Bauraum/Abteil 10 angeordneten Dialysierflüssigkeitsfilter 12 erleichtert, da hierzu lediglich die Zusatztür 16 geöffnet werden muss und die Gehäusetür 8 geschlossen bleiben kann, wodurch die Dialysierflüssigkeitsfilter 12 leicht zugänglich und die in dem Gehäuse 2 untergebrachten anderen Gerätekomponenten weiter, insbesondere vor äußeren Einflüssen, geschützt bleiben.

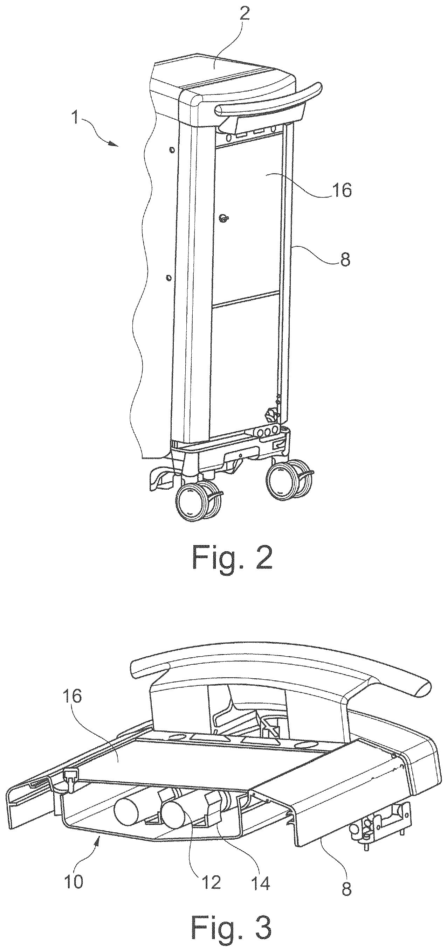

In Figur 2 ist die Ausführungsform des Blutbehandlungsgeräts, vorzugsweise Dialysegeräts 1 gemäß Figur 1 mit geschlossener Gehäusetür 8 und geschlossener Zusatztür 16 dargestellt, wobei die Zusatztür 16 in ihrer geschlossenen Stellung mit der Gehäusetür 8 außen etwa bündig abschließend angeordnet ist.

Wie in Figur 3 dargestellt, ist die Gehäusetür 8 kostengünstig als ein Profil gefertigt, an dem der separat gefertigte Bauraum/Abteil 10 als Zusatzfach (wahlweise) innen oder außen angebracht ist. In einer weiteren nicht dargestellten Ausgestaltung kann der Bauraum/Abteil 10 einstückig mit der Gehäusetür 8 gefertigt sein. Die Gehäusetür 8 und/oder der Bauraum/Abteil 10 kann/können aus Blech oder Kunststoff hergestellt sein. Der Bauraum 10 ist innen und/oder außen etwa bündig mit der Gehäusetür 8 bzw. deren Türrahmen angeordnet.

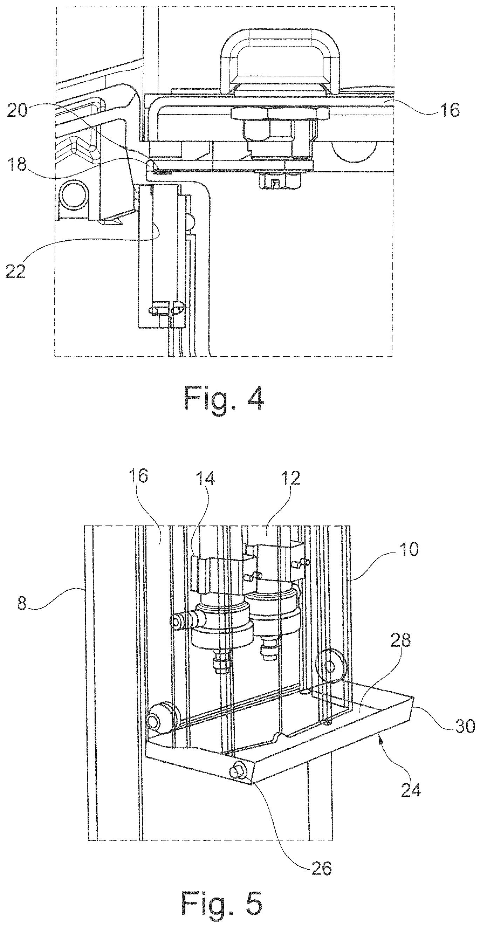

In dem Schnitt in Figur 4 ist gezeigt, wie eine geschlossene Stellung der Zusatztür 16 über eine Schließposition eines Riegels 18 der Zusatztür 16 mittels eines Magneten 20, der am Riegel 18 befestigt ist, und eines Sensors 22 erfassbar ist, der als Positionssensor ausgebildet ist. In geschlossener Stellung der Zusatztür 16 schaltet der Magnet 20 den Sensor 22. Sobald der Magnet 20 vom Sensor 22 entfernt wird, um die Zusatztür 16 zu öffnen, erkennt der Sensor 22 die Veränderung und meldet dem System eine geöffnete Zusatztür 16. Das Sensorsignal kann an eine optische und/oder akustische Warneinrichtung zur Ausgabe einer optischen und/oder akustischen Warnung weitergeleitet werden.

An dieser Stelle sei darauf hingewiesen, dass die vorstehende Beschreibung der sensorischen Erfassung der Schließstellung der Zusatztür 16 nur beispielhaft ist und auch anderweitig erfolgen kann, etwa durch einen mechanisch betätigbaren Positionstaster/Schalter oder dergleichen.

Figur 5 zeigt eine Ansicht von schräg innen mit durchsichtig dargestellten Wänden des Bauraums/Abteils 10. Hier ist in dem Bauraum 10 eine Sammeleinrichtung/Auffangschale 24 zum Sammeln einer Leckagemenge einer Dialysierflüssigkeit, insbesondere aus den Dialysierflüssigkeitsfiltern 12 und/oder daran angeschlossenen Schläuchen, vorgesehen. Die als eine Wanne ausgebildete Sammeleinrichtung 24 hat eine Bodenfläche 28, die den Bauraum 10 nach unten begrenzt. Weiter hat die Sammeleinrichtung 24 eine umlaufende Wandung oder Wannenrand 30, deren Höhe für die zu erwartende Leckagemenge dimensioniert ist.

Mittels eines an der Sammeleinrichtung 24, hier an der Wandung 30, angeordneten Leckagesensors 26 ist die Leckagemenge erfassbar. Das Sensorsignal kann an eine optische und/oder akustische Warneinrichtung zur Ausgabe einer optischen und/oder akustischen Warnung weitergeleitet werden.

Offenbart ist ein Gehäuse für ein Blutbehandlungsgerät wie Dialysegerät, an dem an einer (Gehäuse-)Wand ein, insbesondere nach außen offener, separat zum Gehäuseinneren gehaltener Bauraum/Abteil für eine Gerätekomponente vorgesehen ist. Der Bauraum ist mittels einer Zusatztür individuell verschließbar, wobei die Zusatztür an der Wand angeordnet ist. Im Fall, dass die Wand eine Öffnung aufweist, enthält die Wand eine Gehäusetür, mittels der die Öffnung verschließbar ist, wobei die Zusatztür an der Gehäusetür angeordnet/anscharniert ist. Bei einem erfindungsgemäßen Blutbehandlungsgerät wie Dialysegerät mit dem Gehäuse nach der vorliegenden Erfindung ist die Zusatztür unabhängig von der Gehüsetür bedienbar und somit eine in dem Bauraum/Abteil enthaltene Gerätekomponente zwar mittels der Zusatztür vor Einflüssen von außen geschützt, aber trotzdem beispielsweise für Wartungs- und/oder Bedienzwecke leicht zugänglich.

### Bezugszeichenliste

- 1: Dialysegerät
- 2: Gehäuse
- 4: Wand
- 6: Öffnung
- 8: Gehäusetür
- 10: Bauraum/Abteil
- 12: Gerätekomponente, Dialysierflüssigkeitsfilter
- 14: Halterung
- 16: Zusatztür
- 18: Riegel
- 20: Magnet
- 22: Sensor
- 24: Sammeleinrichtung
- 26: Leckagesensor
- 28: Bodenfläche
- 30: Wandung

## Patentansprüche

1. Gehäuse eines Blutbehandlungsgeräts, mit
wenigstens einer Gehäusewand (4),
einer Öffnung (6), die in der Gehäusewand (4) vorgesehen ist zur Schaffung eines Zugangs in das Gehäuseinnere und
einer Gehäusetür (8), mittels der die Öffnung (6) verschließbar ist,
**dadurch gekennzeichnet, dass**
die Gehäusetür (8) einen separat zum Gehäuseinneren gehaltenen, türinternen Bauraum (10) für wenigstens eine Gerätekomponente (12) aufweist oder ausbildet, der mittels einer Zusatztür (16) verschließbar ist, wobei der Bauraum (10) auch bei geschlossener Gehäusetür (8) von außen über die Zusatztür (16) zugänglich ist, die hierfür von außerhalb des Gehäuses (2) bedienbar ist.

2. Gehäuse nach Anspruch 1, wobei die Gehäusetür (8) und die Zusatztür (16) jeweils unabhängig voneinander, von außen bedienbar sind.

3. Gehäuse nach einem der Ansprüche 1 bis 2, wobei die Zusatztüre (16) an der Gehäusetüre (8) angeordnet ist.

4. Gehäuse nach einem der Ansprüche 1 bis 3, wobei die Gehäusetür (8) und/oder die Zusatztür (16) als eine nach außen öffnende Schwenktür ausgebildet sind/ist.

5. Gehäuse nach einem der Ansprüche 1 bis 4, wobei die Gehäusetür (8) und/oder die Zusatztür (16) als eine Schiebetür ausgebildet ist/sind.

6. Gehäuse nach einem der Ansprüche 1 bis 5, wobei eine offene und/oder eine geschlossene Stellung der Gehäusetür (8) und/oder der Zusatztüre (16) mittels jeweils eines Sensors (22) erfassbar ist.

7. Gehäuse nach einem der Ansprüche 1 bis 6, wobei der Bauraum (10) innen und außen etwa bündig mit der Gehäusetür (8) angeordnet ist.

8. Gehäuse nach einem der Ansprüche 1 bis 7, wobei die Zusatztür (16) in ihrer geschlossenen Stellung mit der Gerätetür (8) außen etwa bündig abschließend angeordnet ist.

9. Gehäuse nach einem der Ansprüche 1 bis 8, wobei in dem Bauraum (10), innerhalb der Gehäusetür (8) eine Sammeleinrichtung (24) zum Sammeln einer Leckagemenge einer Flüssigkeit vorgesehen ist, welche aus der in dem Bauraum (10) untergebrachten Gerätekomponente ausleckt.

10. Gehäuse nach Anspruch 9, wobei eine Leckagemenge mittels eines Leckagesensors (26) erfassbar ist.

11. Gehäuse nach einem der Ansprüche 9 und 10, wobei die Sammeleinrichtung (24) eine Bodenfläche (28) aufweist, die den Bauraum (10) zumindest abschnittsweise begrenzt.

12. Gehäuse nach einem der Ansprüche 1 bis 11, wobei die Gehäusewand (4) eine Rückwand ist.

13. Blutbehandlungsgerät mit einem Gehäuse nach einem der Ansprüche 1 bis 12, wobei in dem Bauraum (10) wenigstens eine Gerätekomponente des Blutbehandlungsgeräts angeordnet ist, die einen Dialysierflüssigkeitsfilter (12), eine Bicarbonatkartusche, und/oder eine Blutdruckmanschette betrifft.

14. Blutbehandlungsgerät nach Anspruch 13, wobei der Bauraum (10) eine Halterung (14) und/oder eine Schnittstelle für die in dem Bauraum (10) enthaltene wenigstens eine Gerätekomponente (12) aufweist.

## Claims

1. Housing of a blood treatment device with
at least one housing wall (4),
one opening (6) which is provided in the housing wall (4) to create an access to the inside of the housing and
one housing door (8) by means of which the opening (6) can be closed,
**characterised in that**
the housing door (8) has or forms an installation space (10) inside the door kept separate from the inside of the housing for at least one device component (12), which can be closed by means of an additional door (16), wherein, even if the housing door (8) is closed, the installation space (10) is accessible from the outside via the additional door (16) which can be operated from outside the housing (2) for this purpose.

2. Housing according to claim 1, wherein the housing door (8) and the additional door (16) can be operated independently of each other in each case from the outside.

3. Housing according to one of the claims 1 to 2, wherein the additional door (16) is arranged on the housing door (8).

4. Housing according to one of the claims 1 to 3, wherein the housing door (8) and/or the additional door (16) is/are realised as a swing door opening to the outside.

5. Housing according to one of the claims 1 to 4, wherein the housing door (8) and/or the additional door (16) is/are realised as a sliding door.

6. Housing according to one of the claims 1 to 5, wherein an open and/or closed position of the housing door (8) and/or the additional door (16) can be detected by means of a sensor (22) in each case.

7. Housing according to one of the claims 1 to 6, wherein the installation space (10) is arranged approximately flush with the housing door (8) on the inside and the outside.

8. Housing according to one of the claims 1 to 7, wherein the additional door (16) is arranged, in its closed position, approximately flush with the device door (8) on the outside.

9. Housing according to one of the claims 1 to 8, wherein in the installation space (10), inside the housing door (8), a collection device (24) is provided for the collection of a leakage volume of a fluid which leaks from the device component integrated in the installation space (10).

10. Housing according to claim 9, wherein a leakage volume can be registered by means of a leakage sensor (26).

11. Housing according to one of the claims 1 to 10, wherein the collection device (24) has a bottom area (28) which confines the installation space (10) at least in sections.

12. Housing according to one of the claims 1 to 11, wherein the housing wall (4) is a rear wall.

13. Blood treatment device with a housing according to one of the claims 1 to 12, wherein in the installation space (10), at least one device component of the blood treatment device is arranged, which concerns a dialysis fluid filter (12), a bicarbonate cartridge and/or a blood pressure cuff.

14. Blood treatment device according to claim 13, wherein the installation space (10) has at least one retainer (14) and/or one interface for the at least one device component (12) contained in the installation space (10).

## Revendications

1. Habillage d'un appareil de traitement du sang, avec
au moins une paroi d'habillage (4),
une ouverture (6), qui est prévue dans la paroi d'habillage (4) en vue de créer un accès à l'intérieur d'habillage, et
une porte d'habillage (8), au moyen de laquelle l'ouverture (6) peut être fermée,
**caractérisé en ce que** la porte d'habillage (8) comporte ou réalise un espace de construction (10), interne à la porte et maintenu séparé de l'intérieur d'habillage, qui est destiné à au moins un composant d'appareil (12) et qui peut être fermé au moyen d'une porte supplémentaire (16),
dans lequel, lorsque la porte d'habillage (8) est fermée, l'espace de construction (10) est aussi accessible de l'extérieur par l'intermédiaire de la porte supplémentaire (16) qui peut être manoeuvrée à cet effet de l'extérieur de l'habillage (2).

2. Habillage selon la revendication 1, dans lequel la porte d'habillage (8) et la porte supplémentaire (16) peuvent être manoeuvrées de l'extérieur, à chaque fois indépendamment l'une de l'autre.

3. Habillage selon l'une quelconque des revendications 1 ou 2, dans lequel la porte supplémentaire (16) est agencée au niveau de la porte d'habillage (8).

4. Habillage selon l'une quelconque des revendications 1 à 3, dans lequel la porte d'habillage (8) et/ou la porte supplémentaire (16) est/sont réalisée(s) comme une porte battante ouvrant vers l'extérieur.

5. Habillage selon l'une quelconque des revendications 1 à 4, dans lequel la porte d'habillage (8) et/ou la porte supplémentaire (16) est/sont réalisée(s) comme une porte coulissante.

6. Habillage selon l'une quelconque des revendications 1 à 5, dans lequel une position ouverte et/ou une position fermée de la porte d'habillage (8) et/ou de la porte supplémentaire (16) peuvent être détectées au moyen d'un capteur (22) à chaque fois.

7. Habillage selon l'une quelconque des revendications 1 à 6, dans lequel l'espace de construction (10) est agencé de manière à affleurer globalement à la porte d'habillage (8) à l'intérieur et à l'extérieur.

8. Habillage selon l'une quelconque des revendications 1 à 7, dans lequel, dans sa position fermée, la porte supplémentaire (16) est agencée de manière à fermer en affleurant globalement à la porte d'habillage (8) à l'extérieur.

9. Habillage selon l'une quelconque des revendications 1 à 8, dans lequel, dans l'espace de construction (10) à l'intérieur de la porte d'habillage (8), il est prévu un dispositif collecteur (24) qui est destiné à collecter une quantité d'un liquide qui fuit d'un composant d'appareil placé dans l'espace de construction (10).

10. Habillage selon la revendication 9, dans lequel une quantité de liquide fuyant peut être détectée au moyen d'un capteur de fuite (26).

11. Habillage selon l'une quelconque des revendications 9 et 10, dans lequel le dispositif collecteur (24) a une surface de fond (28) qui délimite au moins en partie l'espace de construction (10).

12. Habillage selon l'une quelconque des revendications 1 à 11, dans lequel la paroi d'habillage (4) est une paroi arrière.

13. Appareil de traitement du sang avec un habillage selon l'une quelconque des revendications 1 à 12, dans lequel dans l'espace de construction (10) est agencé au moins un composant d'appareil de l'appareil de traitement du sang qui concerne un filtre de liquide de dialyse (12), une cartouche de bicarbonate et/ou un brassard de pression sanguine.

14. Appareil de traitement du sang selon la revendication 13, dans lequel l'espace de construction (10) comporte un support (14) et/ou une interface pour l'au moins un composant d'appareil (12) contenu dans l'espace de construction (10).
